# EUROPÄISCHE PATENTSCHRIFT

(11) **EP 2 892 864 B1**
(45) Veröffentlichungstag und Bekanntmachung des Hinweises auf die Patenterteilung: **25.05.2016**
(21) Anmeldenummer: 13756158.5
(22) Anmeldetag: 02.09.2013
(51) Int. Cl.: C07C 11/24, C01B 3/36, C07C 2/78

(54) **VERFAHREN ZUR HERSTELLUNG VON ACETYLEN UND SYNTHESEGAS**
METHOD FOR PRODUCING ACETYLENE AND SYNTHESIS GAS
PROCÉDÉ DE PRODUCTION D'ACÉTYLÈNE ET DE GAZ DE SYNTHÈSE

(30) Priorität: 05.09.2012 EP 12183175
(43) Veröffentlichungstag der Anmeldung: 15.07.2015
(73) Patentinhaber: BASF SE, 67056 Ludwigshafen am Rhein (DE)
(72) Erfinder: VICARI, Maximilian, 67117 Limburgerhof (DE); WEICHERT, Christian, 67098 Bad Dürkheim (DE); GROßSCHMIDT, Dirk, 68259 Mannheim (DE); RUSS, Michael, 67354 Römerberg (DE); HAIDER, Mirko, 67133 Maxdorf (DE); NEUHAUSER, Horst, 67373 Dudenhofen (DE)
(86) Internationale Anmeldenummer: PCT/EP2013/068102
(87) Internationale Veröffentlichungsnummer: WO 2014/037311

(56) Entgegenhaltungen:
- DE-A1- 1 418 664
- DE-A1- 4 422 815
- US-A- 5 824 834
- H. FRIZ: "Neuere Entwicklungen der Acetylen-Herstellung bei der BASF", CHEMIE INGENIEUR TECHNIK, Bd. 40, Nr. 20, 25. Oktober 1968 (1968-10-25), Seiten 999-1004, XP055085331, ISSN: 0009-286X, DOI: 10.1002/cite.330402005

## Beschreibung

Die vorliegende Erfindung betrifft ein Verfahren zur Herstellung von Acetylen und Synthesegas durch partielle Oxidation von Kohlenwasserstoffen mit Sauerstoff.
Die obige partielle Oxidation ist eine Hochtemperaturreaktion, die üblicherweise in einem Reaktorsystem, umfassend eine Mischeinrichtung, einen Brennerblock sowie eine Quencheinrichtung, durchgeführt wird, und beispielsweise in Ullmanns Encyclopedia of Industrial Chemistry (5th Edition, Volume A1, Seiten 97-144) oder US 005824834A beschrieben ist.
Gemäß Ullmanns Encyclopedia of Industrial Chemistry (Wiley-VCH Verlag, 2008, Acetylene, Seite 13-15) unterscheiden sich die großtechnischen Verfahren zur Herstellung von Acetylen nach dem eingesetzten Quenchmedium, das Wasser oder Öl sein kann.

DE 1418664 zeigt auch ein Verfahren zur Herstellung von Acetylen und Synthesegas durch partielle Oxidation.

Die vorliegende Erfindung betrifft die Prozessvariante, wonach für das schnelle Abkühlen des Spaltgases als Quenchmedium ein Quenchöl eingesetzt wird. Die Aufheizung der Einsatzstoffe erfolgt getrennt in Vorheizern. Die eingesetzten Einsatzstoffe werden in einer Mischeinrichtung gemischt und über einen Mischdiffusor einem Brenner und weiter einem Feuerraum zugeführt. Stromab des Feuerraums wird mittels Düsen ein Quenchöl dem Spaltgas zugeführt und dieses schnell auf etwa 200 - 250 °C abgekühlt. Als Quenchöl wird insbesondere Pyrolyse-Öl eingesetzt. Dies bietet Vorteile bei der Wärmerückgewinnung aus dem Spaltgas, das zur Dampferzeugung genutzt wird.
Mit der beim Quenchen gebildeten Suspension lässt sich der anfallende Ruß ausschleusen. Anschließend wird das regenerierte und gekühlte Quenchöl dem Quenchkreis wieder zugeführt.
Der größte Vorteil gegenüber dem offenen Wasserquench jedoch ist, dass die beschriebene Variante ein zur Umwelt hin geschlossenes Verfahren ermöglicht und damit Kohlenwasserstoffemissionen vermeidet. Der Nachteil dieser Variante ist die Neigung der eingesetzten Öle beim Kontakt mit dem bis hin zu 2000 °C heißen Spaltgas zu vercracken. Dadurch muss der Quenchkreislauf zusätzlich von diesen Crack-Produkten aufwendig gereinigt und das so entfallene Öl ersetzt werden.

Auch bei diesem Verfahren fällt reaktionsbedingt ein größerer Abwasserstrom an, der mit Gasen wie Kohlenmonoxid, Wasserstoff, Acetylen, höheren Acetylenen und BTX-Aromaten gesättigt ist. Bei den höheren Acetylenen handelt es sich vornehmlich um Methyl-, Vinyl-und Diacetylen. Bei den BTX-Aromaten sind vornehmlich Benzol, Toluol, die Xylol-Isomeren, Styrol und Inden.

Ein so verunreinigtes Abwasser kann nicht ohne Vorbehandlung in eine Kläranlage abgegeben werden, da sich z.B. auch im Kanalsystem eine explosionsfähige Atmosphäre durch Ausgasen ausbilden kann. Außerdem belasten die gelösten Kohlenwasserstoff die Abbaurate auch einer adaptierten Kläranlage.

Es war daher Aufgabe der Erfindung, ein Verfahren zur Herstellung von Acetylen und Synthesegas durch partielle Oxydation von Kohlenwasserstoffen zur Verfügung zu stellen, das sowohl eine hohe Ausbeute an Wertprodukt Acetylen als auch die Einhaltung der geltenden Umweltschutzauflagen gewährleistet.

Die Aufgabe wird gelöst durch ein Verfahren zur Herstellung von Acetylen und Synthesegas durch partielle Oxidation von Kohlenwasserstoffen mit Sauerstoff, wobei ein erster Einsatzstrom, enthaltend einen oder mehrere Kohlenwasserstoffe, und ein zweiter, Sauerstoff enthaltender Einsatzstrom,
- getrennt voneinander vorgeheizt,
   in einem Verhältnis der Massenströme des zweiten Einsatzstromes zum ersten Einsatzstrom entsprechend einer Sauerstoffzahl λ von kleiner oder gleich 0,35 gemischt werden, wobei unter Sauerstoffzahl λ das Verhältnis aus der tatsächlich im zweiten Einsatzstrom vorhandenen Sauerstoffmenge zur stöchiometrisch notwendigen Sauerstoffmenge verstanden wird, die für die vollständige Verbrennung des einen oder der mehreren, im ersten Einsatzstrom enthaltenen Kohlenwasserstoffe erforderlich ist,
- über einen Brennerblock einem Feuerraum zugeführt werden, worin die partielle Oxidation der Kohlenwasserstoffe stattfindet,
- unter Erhalt eines Spaltgases, das stromabwärts des Feuerraumes durch Eindüsen eines Quenchöls auf 200 bis 250 °C gequencht wird, wobei
- ein Produktgasstrom I_{g} erhalten wird, der
- in einer Brennerkolonne mit weiterem Quenchöl abgekühlt wird, wobei Flüssigkeit von einer oder mehreren geeigneten Stufen aus der Brennerkolonne abgezogen, durch indirekten Wärmetausch mit Wasser unter Erzeugung von Dampf abgekühlt wird und oberhalb der Stufe, von der er abgezogen wurde, erneut der Brennerkolonne zugeführt wird, unter Erhalt
- eines auf 60°C bis 90°C abgekühlten Produktgasstromes IIg, der
- in einen Schlusskühler geleitet wird, worin durch direkten Wärmetausch mit Wasser ein auf 20°C bis 50°C abgekühlter Produktgasstrom III_{g} erhalten wird, sowie ein Prozesswasserstrom I_{liq} das
- dadurch gekennzeichnet ist, dass der ausgeschleuste Prozesswasserstrom I_{liq} einer Reinigung durch Teilverdampfung in einem einstufigen Entspannungsbehälter unterworfen wird, wobei der Prozesswasserstrom I_{liq} zu einem Anteil von 0,01 Gew. % bis 10 Gew. %, bezogen auf das Gesamtgewicht desselben verdampft wird, unter Erhalt eines gereinigten Prozesswasserstromes Il_{liq}, der in das Abwasser entsorgt wird.

Es wurde gefunden, dass durch eine Teilverdampfung der vereinigten Prozesswasserströme in einem einstufigen Entspannungsbehälter die unerwünschten gelösten Gase, insbesondere polymerisierbare Komponenten, beispielsweise höhere Acetylene, aus den Prozesswasserströmen mit dem Entspannungsdampf in die Gasphase mitgerissen werden, und so weit von der Flüssigphase abgetrennt werden können, dass diese in das überschüssige, anfallende Abwasser entsorgt werden kann.

Die mit dem Entspannungsdampf mitgerissenen Dämpfe von unerwünschten gelösten Gasen können anschließend zum Beispiel nach Kondensation des Wasserdampfes verbrannt oder anderweitig im Prozess entsorgt werden.

Es hat sich überraschend gezeigt, dass eine einstufige Entspannung zur Teilverdampfung des ausgeschleusten Prozesswasserstromes, zu einem Anteil von 0,01 bis 10 Gew.-%, bezogen auf das Gesamtgewicht des ausgeschleusten Prozesswasserstromes eine ausreichende Abreicherung an unerwünschten gelösten Komponenten ermöglicht, so dass das dieses Prozesswasser problemlos und sicher über die Kanalisation in der Kläranlage entsorgt werden kann.

Das Verfahren zur Herstellung von Acetylen und Synthesegas wird erfindungsgemäß mit einer Sauerstoffzahl λ von kleiner oder gleich 0,35 betrieben, wobei unter Sauerstoffzahl λ das Verhältnis aus der tatsächlich im zweiten Einsatzstrom vorhandenen Sauerstoffmenge zur stöchiometrisch notwendigen Sauerstoffmenge verstanden wird, die für die vollständige Verbrennung des einen oder der mehreren, im ersten Einsatzstrom enthaltenen Kohlenwasserstoffe erforderlich ist.

Bei einem Betrieb mit einer Sauerstoffzahl λ im obigen Bereich wird eine hohe Ausbeute an Wertprodukt Acetylen gewährleistet.

Das Verfahren ist unabhängig von der konkreten Ausbildung des Reaktorsystems umfassend Mischeinrichtung, Brennerblock sowie Quencheinrichtung.

Im Folgenden werden die üblicherweise eingesetzten Reaktorsysteme näher erläutert:
Die Ausgangsstoffe, das heißt ein Kohlenwasserstoffe enthaltender Gasstrom, insbesondere Erdgas, und Sauerstoff, werden dabei getrennt aufgeheizt, üblicherweise bis hin zu 600 °C. In einer Mischeinrichtung werden die Reaktanden intensiv vermischt und nach Durchströmen eines Brennerblocks zur exothermen Reaktion gebracht. Der Brennerblock besteht üblicherweise aus einer Vielzahl von parallelen Kanälen, in denen die Strömungsgeschwindigkeit der zündfähigen Sauerstoff/KohlenwasserstoffMischung höher ist als die Flammengeschwindigkeit, um ein Durchschlagen der Flamme in die Mischeinrichtung zu verhindern. Der metallische Brennerblock wird gekühlt, um den thermischen Belastungen standzuhalten. Je nach Aufenthaltszeit in der Mischeinrichtung besteht die Gefahr der Vor- und Rückzündung aufgrund der begrenzten thermischen Stabilität der Mischungen. Hierzu wird der Begriff der Zündverzugszeit, bzw. Induktionszeit gebraucht als die Zeitspanne, in der eine zündfähige Mischung keine nennenswerte intrinsische thermische Veränderung durchläuft. Die Induktionszeit ist abhängig von der Art der eingesetzten Kohlenwasserstoffe, dem Mischungszustand, von Druck und Temperatur. Sie bestimmt die maximale Aufenthaltszeit der Reaktanden in der Mischeinrichtung. Reaktanden wie Wasserstoff, Flüssiggas oder Leichtbenzin, deren Einsatz aufgrund von Ausbeute- und /oder Kapazitätssteigerungen im Syntheseprozess besonders wünschenswert ist, zeichnen sich durch eine vergleichsweise hohe Reaktivität und damit geringe Induktionszeit aus.

Die im derzeitigen Produktionsmaßstab eingesetzten Acetylenbrenner zeichnen sich durch die zylinderförmige Geometrie des Feuerraums aus. Der Brennerblock weist vorzugsweise hexagonal angeordnete Durchführungsbohrungen auf. In einer Ausführungsform sind z.B. 127 Bohrungen ä 27 mm Innendurchmesser hexagonal auf einem kreisförmigen Grundquerschnitt mit Durchmesser von ca. 500 mm angeordnet. In der Regel liegen die eingesetzten Kanaldurchmesser bei etwa 19 bis 27 mm Durchmesser. Der anschließende Feuerraum, in dem die Flamme der Acetylen bildenden partiellen Oxidationsreaktion stabilisiert wird, ist üblicherweise ebenfalls von zylindrischem Querschnitt, ist wassergekühlt und entspricht im Erscheinungsbild dem eines kurzen Rohres (von z.B. 180 bis 533 mm Durchmesser und 380 bis 450 mm Länge). In Höhe des Brennerblocks wird sowohl in axialer als auch in radialer Richtung sogenannter Hilfssauerstoff dem Feuerraum zugeführt. Dadurch wird für eine Flammenstabilisierung und somit für einen definierten Abstand der Flammenwurzel und damit des Reaktionsbeginns zum Reaktionsabbruch durch die Quencheinrichtung gesorgt. Der gesamte Brenner aus Brennerblock und Feuerraum wird in einen Quenchbehälter größeren Querschnitts über einen Flansch von oben eingehängt. Auf Höhe der Austrittsebene aus dem Feuerraum sind am äußeren Umfang derselben Quenchdüsen auf einem oder mehreren Quenchverteilerringen installiert, die das Quenchmedium mit oder ohne Zuhilfenahme eines Zerstäubungsmedium zerstäuben und näherungsweise senkrecht zur Hauptströmungsrichtung der den Feuerraum verlassenden Reaktionsgase eindüsen. Dieser direkte Quench hat die Aufgabe das Reaktionsgemisch extrem schnell abzukühlen, so dass Folgereaktionen, d.h. insbesondere der Abbau von gebildetem Acetylen, eingefroren werden. Die Reichweite und Verteilung der Quenchstrahlen ist dabei idealerweise so bemessen, dass eine möglichst homogene Temperaturverteilung in möglichst kurzer Zeit erreicht wird.

Bei dem vorliegenden technischen Verfahren entsteht neben Acetylen im Wesentlichen Wasserstoff, Kohlenmonoxid und Ruß. Die in der Flammenfront gebildeten Rußpartikel können als Keime an den Feuerraum-Seitenwänden anhaften, worauf es bei geeigneten physikalisch-chemischen Bedingungen zum Aufwachsen, Ablagern und Anbacken von Koksschichten kommt. Diese Ablagerungen werden periodisch im Bereich der Feuerraumwände mittels einer Stocherreinrichtung mechanisch abgereinigt.

Die vorliegende Erfindung nutzt den Umstand, dass beim obigen Verfahren ein Prozesswasserstrom I_{liq} bei einer Temperatur im Bereich zwischen 60 und 96°C, bevorzugt mit einer Temperatur im Bereich von circa 70 bis 80 °C, anfällt. Die enthaltene thermische Energie erlaubt eine ausreichende Abtrennung von unerwünschten gelösten Gasen durch Teilverdampfung ins Vakuum.

Der Produktgasstrom II_{g} wird insbesondere auf 70 °C bis 80 °C abgekühlt.

Im Schlusskühler wird durch direkten Wärmetausch mit Wasser insbesondere ein auf 30 °C bis 40 °C abgekühlter Produktgasstrom III_{g} erhalten.

Bevorzugt erfolgt die Teilverdampfung durch einstufige Entspannung ins Vakuum.

Weiter bevorzugt erfolgt die Teilverdampfung durch einstufige Entspannung adiabat.

In einer Verfahrensvariante kann die Teilverdampfung vorteilhaft durch Wärmeeintrag unterstützt werden.

Eine hinreichende Abtrennung der gelösten Gase kann auch durch eine Strippkolonne erreicht werden. Hierzu werden der vereinigte Prozesswasserstrom auf den Kopf der Kolonne und der Strippdampf im Gegenstrom in den Sumpf der Strippkolonne gegeben. Auch mit diesem Verfahrensschritt wird eine ausreichende Abreicherung der gelösten Gase erreicht. Der apparative Aufwand und somit auch die Investitionskosten des verfahrenstechnischen Schritts sind deutlich höher als beim einfachen, erfindungsgemäßen Flash. Außerdem neigen die Einbauten der dann notwendigen Trennstufen und Verteiler deutlich mehr zum Verschmutzen durch polymerisierende Komponenten als der einfache Aufbau einer einstufigen Entspannung.

Der Entspannungsbehälter ist bevorzugt einstufig und kann mit üblichen Einbauten, wie Packungen oder Böden, wie auch mit einem Demister gegen Tröpfchenmitriss ausgestattet sein.

Möglich ist auch eine mehrstufige Entspannung oder ein Wärmeeintrag im Sumpf wie bei einer Destillationskolonne statt Vorheizen des Zulaufs.

Somit stellt dieses Verfahren eine sehr kostengünstige Möglichkeit der Kreislaufwasserreinigung, bzw. Abwasserreinigung dar.

Das Vakuum kann nach im Stand der Technik bekannter Weise z. B. über eine Dampfstrahlanlage oder einen Wasserringverdichter erzeugt werden. Das Abgas lässt sich dann innerhalb der Anlage weiter behandeln oder auch einer Abgasverbrennung zuführen.

Die Erfindung wird im Folgenden anhand einer Zeichnung sowie eines Ausführungsbeispiels näher erläutert.

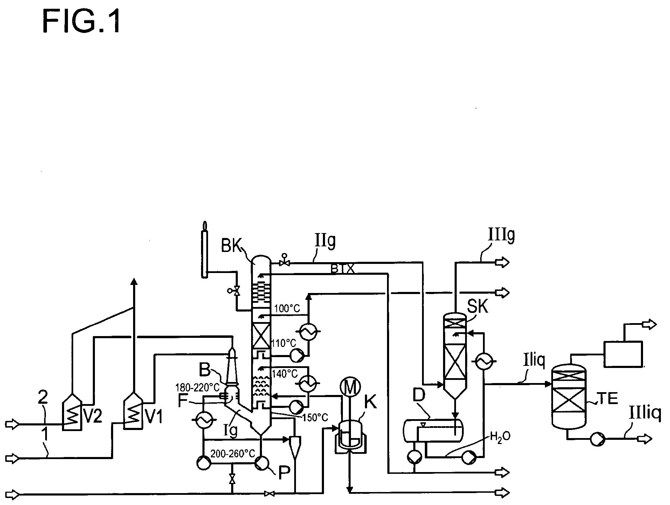

Die einzige Figur 1 zeigt die schematische Darstellung einer bevorzugten erfindungsgemäßen Anlage.

Der in Figur 1 dargestellten Anlage wird ein Kohlenwasserstoffe enthaltender Gasstrom 1 sowie ein Sauerstoff enthaltender Gasstrom 2 zugeführt, über Vorheizer V1 bzw. V2, voneinander getrennt vorgeheizt, über eine Mischeinrichtung und einen Brennerblock B einem Feuerraum F zugeführt, wobei ein Spaltgas erhalten wird, das stromabwärts des Feuerraums F durch Eindüsen eines Quenchöls auf 200 - 250 °C gequencht wird, unter Erhalt eines Produktgasstromes I_{g}, der in einer Brennerkolonne BK, in der in der Figur dargestellten bevorzugten Ausführungsform zweistufig, mit weiterem Quenchöl gekühlt wird, wobei durch Wärmeintegration, durch indirekten Wärmetausch mit Wasser, Dampf erzeugt wird. Aus der Brennerkolonne BK wird auf 80 °C abgekühlter Produktgasstrom II_{g} über Kopf abgezogen, der einem Schlusskühler SK zugeführt wird, worin durch direkten Wärmetausch mit Wasser ein auf 30 °C abgekühlter Produktgasstrom III_{g} erhalten wird, sowie auch ein auszuschleusender Prozesswasserstrom I_{liq} , der einem einstufigen Entspannungsbehälter E zugeführt wird, und worin derselbe teilverdampft wird, unter Erhalt eines gereinigten Prozesswasserstromes II_{liq}, der der Kläranlage zugeführt wird. Um eine Verstopfung der Quenchdüsen zu vermeiden, ist unmittelbar unterhalb der Brennerkolonne BK eine Zerkleinerungspumpe P für den im Quenchöl suspendierten Ruß vorgesehen. Zur Quenchölregenerierung wird ein Teilstrom desselben, einem Rührkessel K zugeführt, der auf 500 °C erhitzt wird, und worin die flüchtigen Anteile verdampfen und reiner Koks am Boden desselben gezogen wird.

Vom Boden des Schlusskühlers SK wird ein Flüssigkeitsstrom abgezogen, der in einem Dekanter D, in eine ölhaltige Fraktion getrennt wird, die insbesondere leichte Aromaten (Benzol/Toloul/Xylol) enthält, und die teilweise ausgeschleust und im Übrigen erneut dem oberen Teil der Brennerkolonne BK zugegeben wird, sowie eine wässrige Fraktion, die größtenteils als Kühlmedium auf den Kopf des Schlusskühlers SK aufgegeben wird und teilweise als überschüssiger Prozesswasserstrom I_{liq} dem einstufigen Entspannungsbehälter E zugeführt wird.

### Ausführungsbeispiel

Ein Prozesswasserstrom I_{liq}, der bei 1.2 bar absolut und 57 °C vorliegt, wird durch Zumischen von Dampf auf 70 °C aufgewärmt und anschließend auf 300 mbar absolut einstufig entspannt. Dabei entstehen 0.62 % Flashdampf bezogen auf den Zulauf. Es ergeben sich folgende Zusammensetzungen und Abreicherungsraten:

| | Abwasser vor Flash [Gew.ppm] | Abwasser nach Flash [Gew.ppm] | Abreicherung [%] |
|---|---|---|---|
| CO | 6.192 | 0.100 | 98.38% |
| Methan | 0.544 | 0.001 | 99.91% |
| Ethan | 0.000 | 0.000 | 100.00% |
| Ethylen | 0.117 | 0.000 | 99.71% |
| Acetylen | 9.190 | 0.232 | 97.47% |
| Propen | 0.006 | 0.000 | 99.75% |
| Propadien | 0.015 | 0.000 | 99.75% |
| Propin | 0.111 | 0.004 | 96.68% |
| Butenin | 0.072 | 0.001 | 97.94% |
| Butadiin | 0.453 | 0.047 | 89.70% |
| Benzol | 536.504 | 27.504 | 94.87% |
| Naphthalin | 0.001 | 0.000 | 94.87% |
| Indan | 28.239 | 3.246 | 88.50% |
| Inden | 104.010 | 13.342 | 87.17% |

Aufgrund der hohen Abreicherung der brennbaren und toxischen Komponenten kann der Abwasserstrom nach dem Entspannen gefahrlos in das Kanalsystem zu einer Kläranlage abgegeben werden.

## Patentansprüche

1. Verfahren zur Herstellung von Acetylen und Synthesegas durch partielle Oxidation von Kohlenwasserstoffen mit Sauerstoff, wobei ein erster Einsatzstrom (1), enthaltend einen oder mehrere Kohlenwasserstoffe, und ein zweiter, Sauerstoff enthaltender Einsatzstrom (2),
- getrennt voneinander vorgeheizt,
- in einem Verhältnis der Massenströme des zweiten Einsatzstromes (2) zum ersten Einsatzstrom (1) entsprechend einer Sauerstoffzahl λ von kleiner oder gleich 0,35 gemischt werden, wobei unter Sauerstoffzahl λ das Verhältnis aus der tatsächlich im zweiten Einsatzstrom (2) vorhandenen Sauerstoffmenge zur stöchiometrisch notwendigen Sauerstoffmenge verstanden wird, die für die vollständige Verbrennung des einen oder der mehreren, im ersten Einsatzstrom (1) enthaltenen Kohlenwasserstoffe erforderlich ist,
- über einen Brennerblock (B) einem Feuerraum (F) zugeführt werden, worin die partielle Oxidation der Kohlenwasserstoffe stattfindet,
- unter Erhalt eines Spaltgases, das stromabwärts des Feuerraumes durch Eindüsen eines Quenchöls auf 200 °C bis 250 °C gequencht wird, wobei
- ein Produktgasstrom I_{g} erhalten wird, der
- in einer Brennerkolonne (BK) mit weiterem Quenchöl abgekühlt wird, wobei Flüssigkeit von einer oder mehreren geeigneten Stufen aus der Brennerkolonne (BK) abgezogen, durch indirekten Wärmetausch mit Wasser unter Erzeugung von Dampf abgekühlt wird und oberhalb der Stufe, von der er abgezogen wurde, erneut der Brennerkolonne (BK) zugeführt wird, unter Erhalt
- eines auf 60 °C bis 90 °C abgekühlten Produktgasstromes II_{g}, der
- in einen Schlusskühler (SK) geleitet wird, worin durch direkten Wärmetausch mit Wasser ein auf 20 °C bis 50 °C abgekühlter Produktgasstrom III_{g} erhalten wird, sowie ein Prozesswasserstrom I_{liq},
- **dadurch gekennzeichnet, dass** der Prozesswasserstrom I_{liq} einer Reinigung durch Teilverdampfung in einem einstufigen Entspannungsbehälter unterworfen wird, wobei der Prozesswasserstrom I_{liq} zu einem Anteil von 0,01 Gew.-% bis 10 Gew.-%, bezogen auf das Gesamtgewicht desselben verdampft wird, unter Erhalt eines gereinigten Prozesswasserstromes II_{liq}, der in das Abwasser entsorgt wird.

2. Verfahren nach Anspruch 1, **dadurch gekennzeichnet, dass** der Produktgasstrom II_{g} auf 70 °C bis 80 °C abgekühlt wird.

3. Verfahren nach Anspruch 1 oder 2, **dadurch gekennzeichnet, dass** im Schlusskühler (SK) durch direkten Wärmetausch mit Wasser ein auf 30 °C bis 40 °C abgekühlter Produktgasstrom III_{g} erhalten wird.

4. Verfahren nach einem der Ansprüche 1 bis 3, **dadurch gekennzeichnet, dass** der Prozesswasserstrom I_{liq} zu einem Anteil von 0,5 Gew.-% bis 2 Gew.-%, bezogen auf das Gesamtgewicht desselben, verdampft wird.

5. Verfahren nach einem der Ansprüche 1 bis 4, **dadurch gekennzeichnet, dass** die Teilverdampfung durch einstufige Entspannung ins Vakuum durchgeführt wird.

6. Verfahren nach Anspruch 5, **dadurch gekennzeichnet, dass** die Teilverdampfung durch einstufige Entspannung adiabat durchgeführt wird.

7. Verfahren nach einem der Ansprüche 1 bis 6, **dadurch gekennzeichnet, dass** die Teilverdampfung durch Wärmeeintrag unterstützt wird.

8. Verfahren nach einem der Ansprüche 1 bis 7, **dadurch gekennzeichnet, dass** der einstufige Entspannungsbehälter (E) mit Einbauten, insbesondere mit Packungen oder Böden, ausgestattet ist.

9. Verfahren nach einem der Ansprüche 1 bis 8, **dadurch gekennzeichnet, dass** der einstufige Entspannungsbehälter (E) mit einem Demister gegen Flüssigkeitströpfchenmitriss ausgestattet ist.

## Claims

1. A process for preparing acetylene and synthesis gas by partial oxidation of hydrocarbons with oxygen, in which a first input stream (1) comprising one or more hydrocarbons and a second input stream (2) comprising oxygen
- are separately preheated,
- mixed in a ratio of the mass flow rates of the second input stream (2) to the first input stream (1) corresponding to an oxygen ratio λ of less than or equal to 0.35, oxygen ratio λ being understood to mean the ratio of the amount of oxygen actually present in the second input stream (2) to the stoichiometrically necessary amount of oxygen required for the complete combustion of the one or more hydrocarbons present in the first input stream (1),
- supplied via a burner block (B) to a combustion chamber (F) in which the partial oxidation of the hydrocarbons takes place,
- to obtain a cracking gas which is quenched to 200°C to 250°C downstream of the combustion chamber by injection of a quench oil, to obtain
- a product gas stream I_{g} which
- is cooled with further quench oil in a burner column (BK), by drawing off liquid from one or more suitable stages in the burner column (BK), cooling it by indirect heat exchange with water to raise steam, and supplying it again to the burner column (BK) above the stage from which it was drawn off,
- to obtain a product gas stream II_{g} which has been cooled to 60°C to 90°C and which
- is passed into a final cooler (SK) in which direct heat exchange with water gives a product gas stream III_{g} cooled to 20°C to 50°C, and a process water stream I_{liq},
- which comprises subjecting the process water stream I_{liq} to a cleaning operation by partial vaporization in a one-stage flash vessel, the process water stream I_{liq} being vaporized in a proportion of 0.01% by weight to 10% by weight, based on the total weight thereof, to obtain a cleaned process water stream II_{liq} which is disposed of in the wastewater.

2. The process according to claim 1, wherein the product gas stream II_{g} is cooled to 70°C to 80°C.

3. The process according to claim 1 or 2, wherein direct heat exchange with water in the final cooler (SK) gives a product gas stream III_{g} cooled to 30°C to 40°C.

4. The process according to any of claims 1 to 3, wherein the process water stream I_{liq} is evaporated in a proportion of 0.5% by weight to 2% by weight, based on the total weight thereof.

5. The process according to any of claims 1 to 4, wherein the partial vaporization is conducted by one-stage flashing into vacuum.

6. The process according to claim 5, wherein the partial vaporization by one-stage flashing is conducted adiabatically.

7. The process according to any of claims 1 to 6, wherein the partial vaporization is promoted by heat input.

8. The process according to any of claims 1 to 7, wherein the one-stage flash vessel (E) is equipped with internals, especially with structured packings or trays.

9. The process according to any of claims 1 to 8, wherein the one-stage flash vessel (E) is equipped with a demister to prevent liquid droplet entrainment.

## Revendications

1. Procédé pour la préparation d'acétylène et de gaz de synthèse par oxydation partielle d'hydrocarbures avec de l'oxygène, un premier flux de départ (1), contenant un ou plusieurs hydrocarbures, et un deuxième flux de départ (2), contenant de l'oxygène étant
- préchauffés séparément l'un de l'autre,
- mélangés dans un rapport massique du deuxième flux de départ (2) au premier flux de départ (1) correspondant à un indice d'oxygène λ inférieur ou égal à 0,35, l'indice d'oxygène λ désignant le rapport de la quantité d'oxygène effectivement présente dans le deuxième flux de départ (2) à la quantité d'oxygène stoechiométriquement nécessaire, qui est nécessaire pour la combustion complète dudit un ou desdits plusieurs hydrocarbures contenus dans le premier flux de départ (1),
- introduits dans un bloc brûleur (B) d'un foyer (F), dans lequel l'oxydation partielle des hydrocarbures a lieu,
- avec obtention d'un gaz de décomposition, qui est refroidi brusquement, en aval du foyer, par injection d'une huile de refroidissement brusque, à 200°C jusqu'à 250°C,
- un flux gazeux produit I_{g} étant obtenu, qui
- est refroidi avec de l'huile de refroidissement brusque supplémentaire dans une colonne de combustion (BK), un liquide étant soutiré d'un ou de plusieurs étages appropriés de la colonne de combustion (BK), refroidi par échange thermique indirect avec de l'eau avec obtention de vapeur et à nouveau introduit dans la colonne de combustion (BK) au dessus de l'étage dont il a été soutiré, avec obtention
- d'un flux gazeux produit II_{g} refroidi à 60°C jusqu'à 90°C, qui
- est guidé dans un refroidisseur final (SK), dans lequel on obtient, par échange thermique direct avec de l'eau, un flux gazeux produit III_{g} refroidi à 20°C jusqu'à 50°C, ainsi qu'un flux d'eau de procédé I_{liq},
**caractérisé en ce que** le flux d'eau de procédé I_{liq} est soumis à une purification par évaporation partielle dans un récipient de détente à un étage, le flux d'eau de procédé I_{liq} étant évaporé à raison d'une proportion de 0,01% en poids à 10% en poids, par rapport au poids total de celui-ci, avec obtention d'un flux d'eau de procédé purifié I_{liq}, qui est évacué dans les eaux usées.

2. Procédé selon la revendication 1, **caractérisé en ce que** le flux gazeux produit II_{g} est refroidi à 70°C jusqu'à 80°C.

3. Procédé selon la revendication 1 ou 2, **caractérisé en ce qu'**on obtient dans le refroidisseur final (SK), par échange thermique direct avec de l'eau, un flux gazeux produit III_{g} refroidi à 30°C jusqu'à 40°C.

4. Procédé selon l'une quelconque des revendications 1 à 3, **caractérisé en ce que** le flux d'eau de procédé I_{liq} est évaporé à raison d'une proportion de 0,5% en poids à 2% en poids, par rapport au poids total de celui-ci.

5. Procédé selon l'une quelconque des revendications 1 à 4, **caractérisé en ce que** l'évaporation partielle est réalisée par une détente à un étage, sous vide.

6. Procédé selon la revendication 5, **caractérisé en ce que** l'évaporation partielle est réalisée par une détente à un étage, de manière adiabatique.

7. Procédé selon l'une quelconque des revendications 1 à 6, **caractérisé en ce que** l'évaporation partielle est soutenue par introduction de chaleur.

8. Procédé selon l'une quelconque des revendications 1 à 7, **caractérisé en ce que** le récipient de détente (E) à un étage est équipé d'encastrements, en particulier de garnissages ou de plateaux.

9. Procédé selon l'une quelconque des revendications 1 à 8, **caractérisé en ce que** le récipient de détente (E) à un étage est équipé d'un dispositif antibuée contre l'entraînement de gouttelettes de liquide.
